# EUROPEAN PATENT SPECIFICATION

(11) **EP 3 321 046 B1**
(45) Date of publication and mention of the grant of the patent: **18.03.2020**
(21) Application number: 15897637.3
(22) Date of filing: 09.07.2015
(51) Int. Cl.: B25J 17/00, B25J 17/02, B25J 9/10, A61B 34/30, A61B 34/00

(54) **TURNING DEVICE AND MEDICAL INSTRUMENT**
DREHVORRICHTUNG UND MEDIZINISCHES INSTRUMENT
DISPOSITIF TOURNANT ET INSTRUMENT MÉDICAL

(43) Date of publication of application: 16.05.2018
(73) Proprietor: Kawasaki Jukogyo Kabushiki Kaisha, Kobe-shi, Hyogo 650-8670 (JP)
(72) Inventor: KAN, Kazutoshi, Hyogo 673-8666 (JP)
(74) Representative: Witte, Weller & Partner Patentanwälte mbB
(86) International application number: PCT/JP2015/003485
(87) International publication number: WO 2017/006374

(56) References cited:
- EP-A1- 2 415 408
- WO-A1-2012/049623
- JP-A- 2008 212 451
- KR-A- 20120 003 091
- US-A1- 2010 160 929
- US-A1- 2010 160 929
- US-A1- 2015 073 434
- US-A1- 2015 073 434

## Description

### Technical Field

The present invention relates to a medical instrument configured by attaching a surgical tool to a tip end of a robot arm, and particularly to a turning device applied to a joint of a robot arm.

### Background Art

In recent years, endoscopic surgical operations (also called laparoscopic surgery) are attracting attention. Used in the endoscopic surgical operations is a medical instrument configured by attaching a surgical tool to a tip end of a shaft. Examples of the surgical tool include forceps, graspers, scissors, staplers, needle holders, and electric scalpels. These surgical tools are used in accordance with contents of operations. In the endoscopic surgical operations, one or a plurality of holes are opened at, for example, an abdominal part of a patient, and a trocar is inserted as a passage port for tools. After that, a laparoscope and a surgical tool are inserted into a coelom through the trocar, and surgery for an affected part of the patient is performed. A surgeon monitors treatments by utilizing a monitor displaying an image of a surgical portion photographed by the laparoscope and operates the medical instrument from an outside of the abdominal part.

PTL 1 describes this type of medical instrument (robotic instrument system). The medical instrument described in PTL 1 includes: a flexible sheath catheter; a guide catheter inserted through the sheath catheter; and a working tool coupled to a distal end of the guide catheter. The working tool is coupled to the distal end of the guide catheter through a turning device. The turning device includes: a first plate provided at the distal end of the guide catheter; a second plate coupled to the working tool; a spherical element sandwiched between the first plate and the second plate; and a plurality of cable-shaped control elements configured to rotate the second plate relative to the first plate. The control elements are inserted through the first plate, and distal ends thereof are coupled to the second plate. Among the plurality of control elements, at least a set of control elements intersect with each other between the first plate and the second plate. By combinations of tensing and relaxing of the plurality of control elements, the second plate is rotated relative to the first plate.

Document US 2015/073434 A1 discloses a rotatable wrist connecting a gripper tool to the distal end of a continuum robot shaft. The rotatable wrist includes a wrist hub that is non-rotatably connected to the distal end of the shaft. A wrist capstan is rotatably connected to the wrist hub and non-rotatably connected to the gripper. A flexible wire loop extends through the wrist hub and partially contacts the wrist capstan. Linear movement of the flexible wire loop through the shaft of the continuum robot causes rotation of the wrist capstan due to friction between the flexible wire loop and the wrist capstan. The wrist also supports selective detachability and control of roll, pitch and roll, pitch yaw and roll.

### Citation List

### Patent Literature

PTL 1: U.S. Patent Application Publication No. US2009/0138025, Figs. 38A-38E, 39A-39C, 40A-40B, and 41A-41B and paragraphs 0235-0246

### Summary of Invention

### Technical Problem

In the turning device of the medical instrument of PTL 1, at least a set of control elements intersect with each other. Since friction is generated between the control elements intersecting with each other, a control characteristic of the control element intersecting with the different control element is different from a control characteristic of the control element not intersecting with any control element. In addition, the control characteristic of the control element intersecting with the different control element changes depending on the degree of influence from the different control element. To be specific, the control characteristic of the control element intersecting with the different control element is not constant and changes. The control of the turning device may become complex by the control element having such complex control characteristic.

The present invention was made under these circumstances, and an object of the present invention is to solve at least one of problems of conventional arts.

### Solution to Problem

A turning device according to one aspect of the present invention is a turning device provided at a joint of a robot arm, the turning device including: a shaft cover including a tubular wall extending in an axial direction; a turning shaft inserted into an inside of the wall of the shaft cover and extending in the axial direction; a bearing causing the shaft cover to support the tuning shaft such that the turning shaft is rotatable about a center axis of the turning shaft; and at least one operating wire coupled to the turning shaft at the inside of the wall of the shaft cover, wherein: the shaft cover includes a guide guiding the at least one operating wire; the guide is formed to be symmetrical about a symmetry plane passing through a center axis of the shaft cover and parallel to the axial direction; and each of portions of the guide which portions are located at both respective sides of the symmetry plane includes an outer peripheral portion formed on an outer surface of the wall and extending from a base end of the wall in the axial direction, an inner peripheral portion formed along an inner surface of the wall and extending in a circumferential direction, and a connecting portion connecting the outer peripheral portion and the inner peripheral portion.

A medical instrument according to another aspect of the present invention includes: a robot arm including the turning device; and an end effector provided at a tip end of the robot arm.

According to the above turning device and medical instrument, since the operating wire is guided by the guide of the shaft cover, a part of the operating wire and another part of the operating wire do not interfere with each other, or a plurality of operating wires do not interfere with one another. Therefore, it is possible to avoid a case where the control characteristic of the operating wire changes by the interference between the parts of the operating wire or between the operating wires.

### Advantageous Effects of Invention

The present invention can provide a medical instrument and a robot arm of the medical instrument, by each of which at least one of problems of conventional arts is solved.

### Brief Description of Drawings

Fig. 1 is a diagram showing a schematic configuration of a medical instrument according to one embodiment of the present invention.
Fig. 2 is a partially enlarged sectional view of the medical instrument and shows a schematic configuration of a wrist joint.
Fig. 3 is a side view of a shaft cover.
Fig. 4 is a sectional view showing a schematic configuration of a turning device.
Fig. 5 is a diagram when viewed from a direction indicated by an arrow V of Fig. 3.
Fig. 6 is a sectional view taken along line VI-VI of Fig. 3.
Fig. 7 is a partially enlarged sectional view of the medical instrument according to Modified Example 1 and shows a schematic configuration of the wrist joint of the medical instrument.
Fig. 8 is a diagram showing one example of an interlock mechanism included in the turning device.

### Description of Embodiments

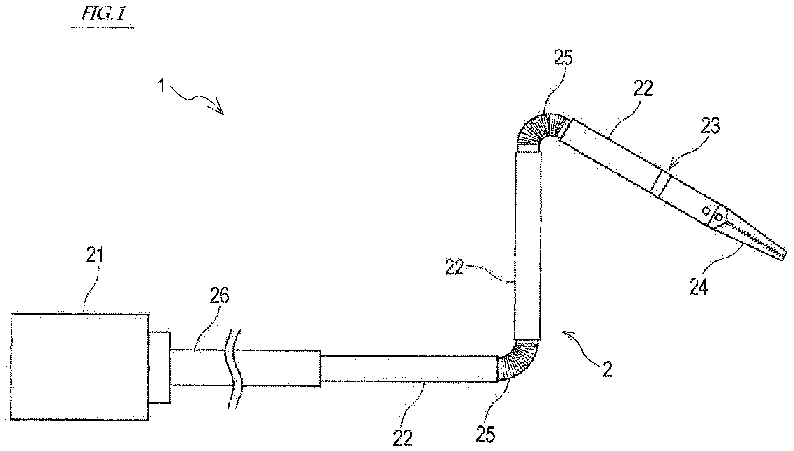

Hereinafter, an embodiment of the present invention will be explained in reference to the drawings. Fig. 1 is a diagram showing a schematic configuration of a medical instrument 1 according to one embodiment of the present invention. As shown in Fig. 1, the medical instrument 1 is a so-called medical manipulator and includes: a robot arm 2 having a thin and long shaft shape; and an end effector 24 coupled to a tip end of the robot arm 2. The robot arm 2 includes: a base 21 provided at a base end of the robot arm 2; a wrist joint 23 provided at a tip end of the robot arm 2; a soft shaft 26 coupled to the base 21; a plurality of links 22 provided between the soft shaft 26 and the wrist joint 23; and intermediate joints 25 coupling the plurality of links 22 to one another.

The base 21 is connected to a moving mechanism or a drive mechanism (both not shown). The soft shaft 26 is coupled to the base 21 so as to be rotatable. The soft shaft 26 has flexibility and a torque transmitting property of transmitting input torque to the link 22 located at the base end of the robot arm 2 among the plurality of links 22. The plurality of links 22 are hard tubular members, and wires and cables are inserted into insides of the links 22. The wrist joint 23 couples the end effector 24 to the link 22 located at the tip end of the robot arm 2 among the plurality of links 22 such that the end effector 24 is rotatable.

The end effector 24 denotes an actual operated portion which is inserted into a surgical portion of an abdominal cavity of a patient and can be driven from an outside of the abdominal cavity to execute a desired treatment or medical function with respect to a target tissue of the surgical portion. The end effector 24 may be a surgical tool such as forceps, a grasper, scissors, a stapler, a needle holder, or an electric scalpel. Or, the end effector 24 may be an electrically driven instrument, such as an electrosurgical electrode, a transducer, or a sensor. Or, the end effector 24 may be a suction nozzle, a gas injecting nozzle, a washing nozzle, a treatment fluid nozzle, an accessory introducing nozzle, or a nozzle through which a fluid for biopsy removal or the like is supplied. Or, the end effector 24 may be provided with an image pickup apparatus such as a camera.

The medical instrument 1 denotes a medical instrument including the end effector 24. The medical instrument 1 may be directly operated by a surgeon or may be operated by remote control by a remote surgical system using a robot or the like.

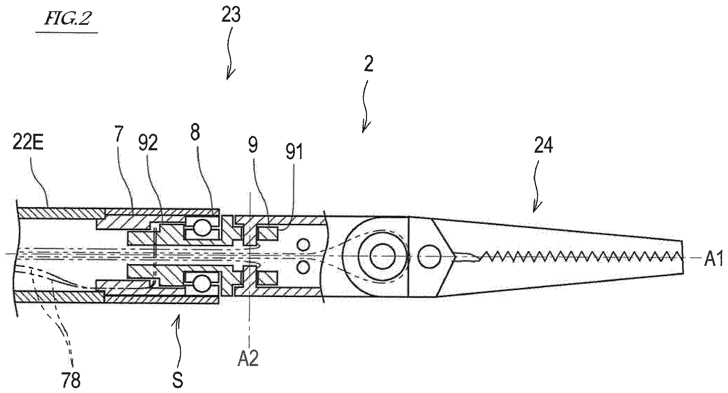

Next, the wrist joint 23 included in the medical instrument 1 will be explained in detail. Fig. 2 is a partially enlarged sectional view of the medical instrument 1 and shows a schematic configuration of the wrist joint 23.

As shown in Fig. 2, the wrist joint 23 couples the end effector 24 to the link 22 located at a most tip end side among the links 22 included in the robot arm 2 (this link 22 is hereinafter referred to as a "tip end link 22E"). The end effector 24 is rotatable relative to the tip end link 22E about a first axis A1 (turning axis) of the wrist joint 23. Further, the end effector 24 is rotatable relative to the tip end link 22E about a second axis A2 (swing axis) of the wrist joint 23, the second axis A2 being perpendicular to the first axis A1. Hereinafter, a direction parallel to a direction in which the first axis A1 extends is referred to as an "axial direction Da."

The wrist joint 23 is provided with a turning device S substantially constituted by: a shaft cover 7 coupled to the tip end link 22E; a turning shaft 9 coupled to the end effector 24; a bearing 8 supporting the turning shaft 9 such that the turning shaft 9 is rotatable relative to the shaft cover 7 about the first axis A1; and a drive mechanism 10 configured to rotate the turning shaft 9 relative to the shaft cover 7.

The turning shaft 9 is a tubular shaft member extending in the axial direction Da, and the first axis A1 passes through a center axis of the turning shaft 9. A plurality of wires and cables such as sensor cables are inserted through the turning shaft 9. Examples of the wires inserted through the turning shaft 9 include: an operating wire by which the end effector 24 is operated; an operating wire by which the end effector 24 is rotated around the second axis A2; and a sensor cable of a sensor provided at the end effector 24.

A tip end portion 91 of the turning shaft 9 is joined to a base portion of the end effector 24 by a pin. The type of the joining by the pin is not limited, but the joining by the pin can be realized by, for example, a pin projecting from the base portion of the end effector 24 in a direction parallel to the second axis A2; and a supporting hole supporting the pin and formed at the tip end portion 91 of the turning shaft 9.

A base portion 92 of the turning shaft 9 is inserted into the shaft cover 7. A stepped surface 93 is formed on an outer surface of the base portion 92 of the turning shaft 9 by a difference between outer diameters of the turning shaft 9. The bearing 8 is provided between the tip end portion 91 and base portion 92 of the turning shaft 9 in the axial direction Da.

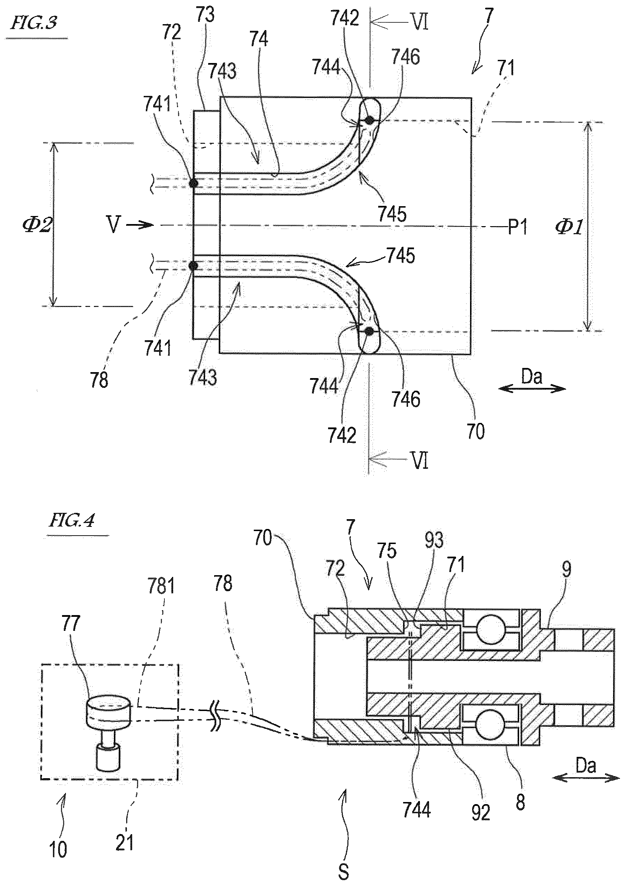

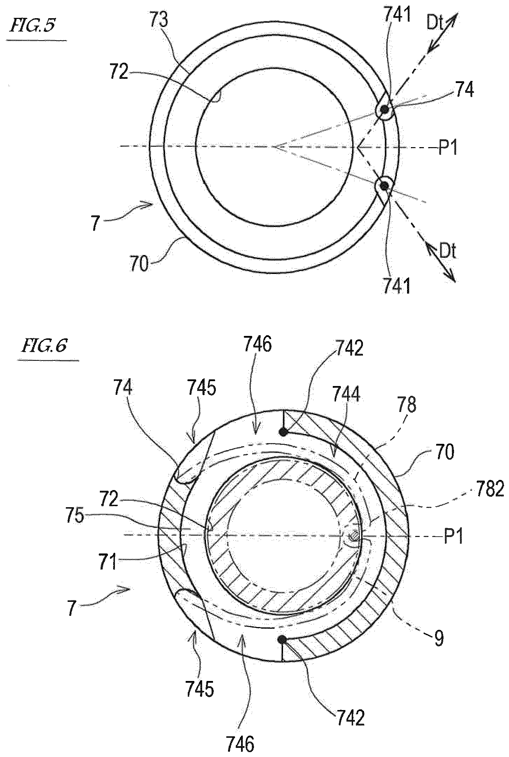

Fig. 3 is a side view of the shaft cover 7. Fig. 4 is a sectional view showing a schematic configuration of the turning device S. Fig. 5 is a diagram when viewed from a direction indicated by an arrow V of Fig. 3. Fig. 6 is a sectional view taken along line VI-VI of Fig. 3. In Fig. 3, an operating wire 78 provided at a guide 74 of the shaft cover 7 is shown by light black paint and two-dot chain lines (virtual lines). Further, in Fig. 6, the operating wire 78 provided at the guide 74 of the shaft cover 7 is shown by light black paint and two-dot chain lines, and the turning shaft 9 inserted into the shaft cover 7 is shown by two-dot chain lines.

As shown in Figs. 2 to 6, the shaft cover 7 includes a tubular wall 70 extending in the axial direction Da. A cutout 73 that is continuous in a circumferential direction is provided at a base end edge of an outer surface of the wall 70. The shaft cover 7 and the tip end link 22E are coupled to each other by utilizing the cutout 73.

The shaft cover 7 is formed to be symmetrical about a symmetry plane P1 passing through a center axis of the shaft cover 7 and parallel to the axial direction Da. The guide 74 guiding the operating wire 78 is formed at the shaft cover 7. The guide 74 is formed to be symmetrical about the symmetry plane P1 at both sides of the symmetry plane P1.

The guide 74 forms a wiring route for the operating wire 78 at the shaft cover 7, and one operating wire 78 is provided at one guide 74. The operating wire 78 provided at the guide 74 is a wire by which the turning shaft 9 is turned. The operating wire 78 according to the present embodiment is an endless wire and is wound around the turning shaft 9 and a pulley 77 provided at the base 21. A part of the operating wire 78 which part is located between the turning shaft 9 and the pulley 77 extends through insides of the links 22. The operating wire 78 is wound around an outer peripheral surface of the turning shaft 9 in a circumferential direction and is fixed to the turning shaft 9 on the outer peripheral surface of the turning shaft 9. The pulley 77 is rotated forward and backward by a driving source (not shown). An operation of the driving source is controlled by a control device (not shown). By the forward and backward rotations of the pulley 77, the operating wire 78 is tensed or relaxed, and therefore, the turning shaft 9 is rotated about the first axis A1.

A method of fixing the operating wire 78 to the turning shaft 9 is not limited. For example, a part of the operating wire 78 can be fixed to the turning shaft 9 by: a recess or hole provided on the outer peripheral surface of the turning shaft 9; and a pin serving as a retainer for the part of the operating wire 78, the part being fitted in this recess or hole in the form of a loop. To increase a rotation angle range of the turning shaft 9, it is desirable that a fixing width (circumferential width) of the operating wire 78 fixed to the turning shaft 9 be smaller. It should be noted that the operating wire 78 does not have to be endless and may be divided into plural parts. For example, when the operating wire 78 is cut on the outer peripheral surface of the turning shaft 9, one of end portions of the operating wire 78 and the other end portion may be arranged close to each other on the outer surface of the turning shaft 9 and coupled to the turning shaft 9. Further, for example, when a part of the operating wire 78 which part is wound around the pulley 77 is cut, a plurality of pulleys 77 may be provided, and cut end portions of the operating wire 78 may be wound around the respective pulleys 77. Furthermore, a plurality of operating wires 78 may be wound around one turning shaft 9. In this case, the shaft cover 7 is provided with the guides 74, the number of which corresponds to the number of operating wires 78.

Each of portions of the guide 74 which portions are located at both respective sides of the symmetry plane P1 includes: an outer peripheral portion 743 formed on the outer surface of the wall 70 of the shaft cover 7; an inner peripheral portion 744 formed along an inner surface of the wall 70; and a connecting portion 745 smoothly connecting the outer peripheral portion 743 and the inner peripheral portion 744.

The outer peripheral portions 743 of the guide 74 are grooves (i.e., continuous recesses) formed on the outer surface of the wall 70. Each of the outer peripheral portions 743 includes a starting point 741 that is a base end of the wall 70. The outer peripheral portion 743 extends in the axial direction Da from the starting point 741 to an axial-direction-Da intermediate portion of the wall 70.

As shown in Fig. 5, when the shaft cover 7 is viewed from the axial direction Da, a center angle between the starting point 741 at one side of the symmetry plane P1 and the starting point 741 at the other side of the symmetry plane P1 is about 45°. Further, depth directions Dt of the grooves of the outer peripheral portion 743 do not coincide with a radial direction of the shaft cover 7 and is inclined relative to the radial direction. With this, a deeper groove can be formed on a wall having a limited thickness. The depth direction Dt of the outer peripheral portion 743 at one side of the symmetry plane P1 and the depth direction Dt of the other outer peripheral portion 743 at the other side of the symmetry plane P1 intersect with each other on the symmetry plane P1.

The inner peripheral portions 744 of the guide 74 are formed by cooperation of the shaft cover 7 and the turning shaft 9. A large-diameter portion 71 having a first inner diameter Φ1, a small-diameter portion 72 having a second inner diameter Φ2, and a stepped surface 75 formed by a difference between the inner diameters of the large-diameter portion 71 and the small-diameter portion 72 are formed on the inner surface of the wall 70 of the shaft cover 7. The second inner diameter Φ2 is smaller than the first inner diameter Φ1, and the large-diameter portion 71 is located at a tip end side of the small-diameter portion 72.

The stepped surface 75 of the shaft cover 7 and the stepped surface 93 of the turning shaft 9 face each other with an interval therebetween in the axial direction Da. Then, an annular space surrounded by the inner surface of the shaft cover 7 and the outer surface of the turning shaft 9 is formed between the stepped surfaces 75 and 93. At least a part of this annular space is utilized as the inner peripheral portions 744 of the guide 74. The inner peripheral portions 744 extend in the circumferential direction along the inner surface of the wall 70. In other words, the inner peripheral portions 744 of the guide 74 extend in the circumferential direction along the outer surface of the turning shaft 9. It should be noted that the annular space located between the two stepped surfaces 75 and 93 and surrounded by the inner surface of the shaft cover 7 and the outer surface of the turning shaft 9 is utilized as the inner peripheral portions 744 of the guide 74.

Each of the connecting portions 745 of the guide 74 is formed by: a communication hole 746 penetrating an inside and outside of the wall 70; and a groove (i.e., a continuous recess) connecting the communication hole 746 and the corresponding outer peripheral portion 743 and formed on the outer surface of the wall 70. The communication hole 746 extends from an opening end 742 in a tangential direction (circumferential direction) of the opening end 742, the opening end 742 being located at a part of the large-diameter portion 71 which part is located right next to a boundary between the large-diameter portion 71 and the small-diameter portion 72. The inner peripheral portion 744 of the guide 74 exists inside the communication hole 746. It should be noted that in the guide 74, a center angle between the opening end 742 located at one side of the symmetry plane P1 and the opening end 742 located at the other side of the symmetry plane P1 is about 180° (see Fig. 6).

The groove of the connecting portion 745 which groove connects the communication hole 746 and the outer peripheral portion 743 forms a circular-arc curved line to gently connect the outer peripheral portion 743 extending in the axial direction Da and the inner peripheral portion 744 extending in the circumferential direction. A curvature radius of the connecting portion 745 is adequately large to such an extent that the operating wire 78 can smoothly pass through the connecting portion 745.

The operating wire 78 provided at the guide 74 of the shaft cover 7 configured as above extends through the outer peripheral portions 743 in the axial direction Da and extends through the inner peripheral portions 744 in the circumferential direction. Further, in the connecting portions 745, an extending direction of the operating wire 78 gradually changes from the axial direction Da to the circumferential direction.

According to the operating wire 78 provided at the guide 74 of the shaft cover 7 as above, a part of the operating wire 78 does not interfere with another part of the operating wire 78. To be specific, the operating wire 78 does not intersect or overlap at the wrist joint 23.

As explained above, the turning device S of the present embodiment includes: the shaft cover 7 including the tubular wall 70 extending in the axial direction Da; the turning shaft 9 inserted into the wall 70 of the shaft cover 7 and extending in the axial direction Da; the bearing 8 causing the shaft cover 7 to support the tuning shaft 9 such that the turning shaft 9 is rotatable about the center axis of the turning shaft 9; and the operating wire 78 coupled to the turning shaft 9 inside the wall 70 of the shaft cover 7. The shaft cover 7 includes the guide 74 guiding the operating wire 78. The guide 74 is formed to be symmetrical about the symmetry plane P1 passing through the center axis of the shaft cover 7 and parallel to the axial direction Da. Each of portions of the guide 74 which portions are located at both respective sides of the symmetry plane P1 includes: the outer peripheral portion 743 formed on the outer surface of the wall 70 and extending in the axial direction Da from the base end of the wall 70; the inner peripheral portion 744 formed along the inner surface of the wall 70 and extending in the circumferential direction; and the connecting portion 745 connecting the outer peripheral portion 743 and the inner peripheral portion 744.

In the turning device S configured as above, an action direction of tensile force acting on the operating wire 78 at a pulled and tensed part of the operating wire 78 provided at the guide 74 changes within the wiring route extending from the starting point 741 of the guide 74 to the outer surface of the turning shaft 9. The degree of a change in the action direction of the tensile force acting on the operating wire 78 is reduced by providing the operating wire 78 along the guide 74. To be specific, since the operating wire 78 is guided by the guide 74, a rapid change in the action direction of the tensile force acting on the operating wire 78 is avoided. Therefore, it is possible to avoid a case where the operating wire 78 partially receives an excess load or twists.

In the turning device S configured as above, the guide 74 of the shaft cover 7 continuously extends as a single line from the starting point 741 located at one side of the symmetry plane P1 to the starting point 741 located at the other side of the symmetry plane P1 and does not overlap or intersect. Therefore, a part of the operating wire 78 provided at the guide 74 and another part of the operating wire 78 do not intersect with each other. It should be noted that when a plurality of operating wires 78 are provided, the guides 74 which are independent from one another and do not overlap or intersect with one another are formed on the shaft cover 7 for the respective operating wires 78. As above, the operating wire 78 is guided at the shaft cover 7 such that: a part of the operating wire 78 and another part of the operating wire 78 do not intersect with each other; and a plurality of operating wires 78 do not intersect with one another. Therefore, it is possible to avoid a case where the control characteristic of the operating wire 78 changes by, for example, frictional force generated by interference between the operating wires 78 or between parts of the operating wire 78.

Further, in the turning device S of the present embodiment, each of the connecting portions 745 is formed by: the communication hole 746 penetrating the inside and outside of the wall 70 and opening on the corresponding inner peripheral portion 744; and the groove connecting the communication hole 746 and the corresponding outer peripheral portion 743 in the form of the curved line and provided on the outer surface of the wall 70.

With this, a part of the operating wire 78 which part is guided by the outer peripheral portion 743 and extending in the axial direction Da and another part of the operating wire 78 which part is guided by the inner peripheral portion 744 and extending in the circumferential direction are gently (smoothly) coupled to each other by yet another part of the operating wire 78 which part is guided by the connecting portion 745. Therefore, it is possible to avoid a case where the operating wire 78 partially receives an excessive load, breaks, or twists.

In the turning device S of the present embodiment, the communication hole 746 of the connecting portion 745 extends from the opening end 742 in the tangential direction of the opening end 742. With this, a part of the operating wire 78 which part passes through the communication hole 746 extends in the tangential direction of the opening end 742 and is further smoothly connected to a part of the operating wire 78 which part is guided by the inner peripheral portion 744.

In the turning device S of the present embodiment, the annular first stepped surface 75 facing one side in the axial direction Da is formed on the inner surface of the wall 70 of the shaft cover 7, and the annular second stepped surface 93 facing the other side in the axial direction Da is formed on the outer surface of the turning shaft 9. These stepped surfaces 75 and 93 are arranged so as to face each other with an interval therebetween in the axial direction Da. The inner peripheral portions 744 of the guide 74 are formed between the stepped surfaces 75 and 93 by the inner surface of the shaft cover 7 and the outer surface of the turning shaft 9.

With this, although it is difficult to subject the inner surface of the wall 70 of the shaft cover 7 to machine work, the inner peripheral portions 744 of the guide 74 can be formed on the inner surface of the wall 70 without forming grooves by machine work.

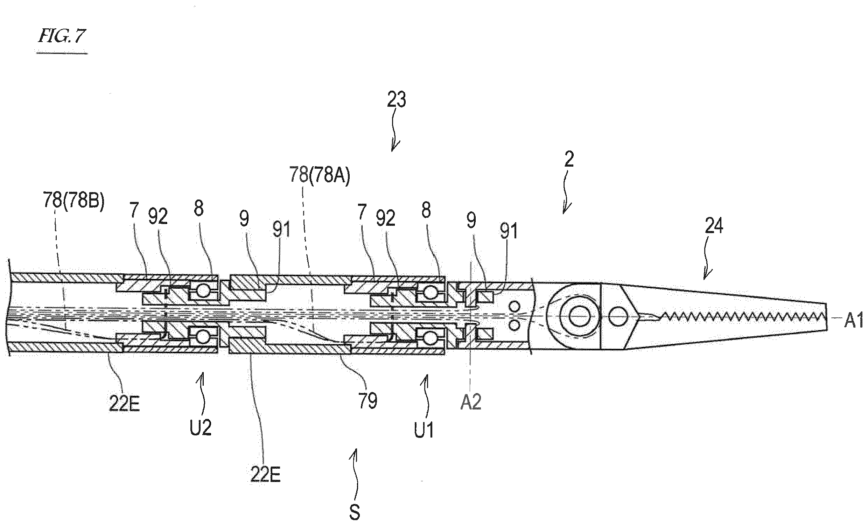

Next, Modified Example 1 of the above embodiment will be explained. Fig. 7 is a partially enlarged sectional view of the medical instrument 1 according to Modified Example 1 and shows a schematic configuration of the wrist joint 23 of the medical instrument 1. In the explanations of the present modified example, the same reference signs are used for the same or corresponding members as the above embodiment, and a repetition of the same explanation may be avoided.

As shown in Fig. 7, the wrist joint 23 of the medical instrument 1 according to Modified Example 1 is provided with the turning device S including a plurality of turning units coupled to one another in the axial direction Da, each turning unit including the turning shaft 9, the shaft cover 7, the bearing 8, and the operating wire 78. In the turning device S according to the present modified example, two turning units U1 and U2 are coupled to each other through a coupling member 79 (or directly coupled to each other).

Regarding the turning unit U1 arranged at the tip end side out of the two turning units U1 and U2, the turning shaft 9 is coupled to the end effector 24, and the shaft cover 7 is coupled to the tubular coupling member 79. Regarding the turning unit U2 arranged at the base portion side out of the two turning units U1 and U2, the turning shaft 9 is coupled to the coupling member 79, and the shaft cover 7 is coupled to the tip end link 22E.

A winding range of the operating wire 78 around the turning shaft 9 is less than a range corresponding to one circumference of the turning shaft 9. Therefore, when the turning device S includes one turning unit, a rotation range of the turning shaft 9 is less than 360°. As compared to this, when the turning device S includes a plurality of turning units U1 and U2 arranged in series as in the present modified example, the rotation range of the turning shaft 9 can be increased.

In the wrist joint 23 of the medical instrument 1 according to the present modified example, the operating wire 78 (78A) of the turning unit U1 and the operating wire 78 (78B) of the turning unit U2 can be directly or indirectly coupled to each other, so that the turning unit U1 and the turning unit U2 can be operated in conjunction with each other. When the turning unit U1 and the turning unit U2 are operated in conjunction with each other, the operating wire 78A of the turning unit U1 and the operating wire 78B of the turning unit U2 move at the same distance and speed.

Further, the operating wire 78A of the turning unit U1 and the operating wire 78B of the turning unit U2 can be uncoupled from each other. By uncoupling the operating wires 78A and 78B from each other, the turning unit U1 and the turning unit U2 can be operated independently.

When the turning unit U1 and the turning unit U2 are operated in conjunction with each other, the rotation of the end effector 24 (the turning shaft 9 of the turning unit U1) can be made higher than when the turning unit U1 and the turning unit U2 are operated independently. To be specific, when the operating wires 78 are tensed at the same distance and speed, the rotation amount of the end effector 24 in a case where the turning units U1 and U2 are operated in conjunction with each other is larger than that in a case where the turning units U1 and U2 are operated independently.

Since it is possible to select whether to operate the turning units U1 and U2 in conjunction with each other or operate the turning units U1 and U2 independently, the turning of the end effector 24 around the first axis A1 can be controlled more finely.

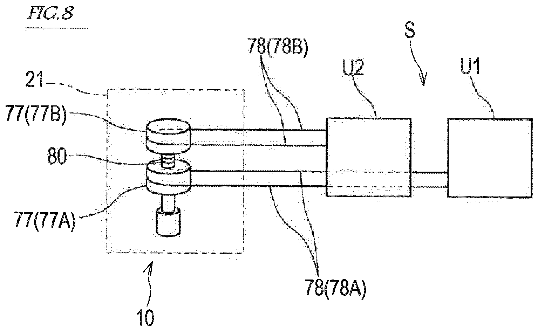

It should be noted that the turning device S according to Modified Example 1 includes an interlock mechanism configured to switch between a state where the turning units U1 and U2 are operated in conjunction with each other and a case where the turning units U1 and U2 are operated independently. The interlock mechanism is, for example, a mechanism configured to perform coupling and uncoupling of the operating wires of a plurality of turning units, the operating wires being operable at the same time. The interlock mechanism according to the present modified example can perform the coupling and uncoupling of the operating wire 78A of the turning unit U1 and the operating wire 78B of the turning unit U2. For example, as shown in Fig. 8, the interlock mechanism can be realized such that: a pulley 77A around which the operating wire 78A of the turning unit U1 is wound and a pulley 77B around which the operating wire 78B of the turning unit U2 is wound are arranged coaxially; and an engaging/disengaging device 80, such as a clutch, configured to switch between engagement and disengagement of the pulleys 77A and 77B arranged coaxially is provided.

The foregoing has explained the preferred embodiment of the present invention and the modified examples. The above configuration can be changed as below, for example. For example, although the turning device S is applied to the wrist joint 23, the turning device S may be applied to the intermediate joint 25.

From the foregoing explanation, many modifications and other embodiments of the present invention are obvious to one skilled in the art. Therefore, the foregoing explanation should be interpreted only as an example and is provided for the purpose of teaching the best mode for carrying out the present invention to one skilled in the art. The structures and/or functional details may be modified within the scope of the present invention, as defined by the appended claims.

### Reference Signs List

- 1: medical instrument
- 2: robot arm
- 7: shaft cover
- 71: large-diameter portion
- 72: small-diameter portion
- 73: cutout
- 74: guide
- 741: starting point
- 742: opening end
- 743: outer peripheral portion
- 744: inner peripheral portion
- 745: connecting portion
- 746: communication hole
- 75: stepped surface
- 8: bearing
- 9: turning shaft
- 10: drive mechanism
- 21: base
- 22, 22E: link
- 23: wrist joint
- 24: end effector
- 25: intermediate joint
- 26: soft shaft
- 77: pulley
- 78 (78A, 78B): operating wire
- 79: coupling member
- 80: engaging/disengaging device
- A1: first axis
- A2: second axis
- S: turning device
- U1, U2: turning unit

## Claims

1. A turning device (S) provided at a joint of a robot arm (2),
the turning device (S) comprising:
a shaft cover (7) including a tubular wall (70) extending in an axial direction (Da);
a turning shaft (9) inserted into an inside of the wall (70) of the shaft cover (7) and extending in the axial direction (Da);
a bearing (8) causing the shaft cover (7) to support the turning shaft (9) such that the turning shaft (9) is rotatable about a center axis of the turning shaft (9); and
at least one operating wire (78) coupled to the turning shaft (9) at the inside of the wall (70) of the shaft cover (7), wherein:
the shaft cover (7) includes a guide (74) guiding the at least one operating wire (78);
the guide (74) is formed to be symmetrical about a symmetry plane (P1) passing through a center axis of the shaft cover (7) and parallel to the axial direction (Da); and
each of portions of the guide (74) which portions are located at both respective sides of the symmetry plane (P1) includes
an outer peripheral portion (743) formed on an outer surface of the wall (70) and extending from a base end of the wall (70) in the axial direction (Da),
an inner peripheral portion (744) formed along an inner surface of the wall (70) and extending in a circumferential direction, and
a connecting portion (745) connecting the outer peripheral portion (743) and the inner peripheral portion (744).

2. The turning device (S) according to claim 1, wherein the connecting portion (745) is formed by:
a communication hole (746) penetrating an inside and outside of the wall (70) and opening on the inner peripheral portion (744); and
a groove connecting the communication hole (746) and the outer peripheral portion (743) in a form of a curved line and provided on the outer surface of the wall (70).

3. The turning device (S) according to claim 2, wherein the communication hole (746) extends from an opening end of the communication hole (746) in a tangential direction of the opening end.

4. The turning device (S) according to any one of claims 1 to 3, wherein:
the inner surface of the wall (70) of the shaft cover (7) includes an annular first stepped surface (75) facing one side in the axial direction (Da);
an outer surface of the turning shaft (9) includes an annular second stepped surface (93) facing the other side in the axial direction (Da);
the first stepped surface (75) and the second stepped surface (93) are arranged so as to face each other with an interval therebetween in the axial direction (Da); and
the inner peripheral portion (744) is formed between the first stepped surface (75) and the second stepped surface (93) by an inner surface of the shaft cover (7) and the outer surface of the turning shaft (9).

5. The turning device (S) according to any one of claims 1 to 4, comprising a plurality of turning units (U1,U2) coupled to one another in the axial direction (Da), each of the turning units (U1,U2) including the turning shaft (9), the shaft cover (7), the bearing (8), and the at least one operating wire (78).

6. The turning device (S) according to claim 5, further comprising an interlock mechanism configured to couple the operating wires (78) of the plurality of turning units (U1,U2) to one another to operate the plurality of turning units (U1,U2) in conjunction with one another.

7. A medical instrument (1) comprising:
a robot arm (2) including the turning device (S) according to any one of claims 1 to 6; and
an end effector (24) provided at a tip end of the robot arm (2).

## Patentansprüche

1. Drehvorrichtung (S), die am Gelenk eines Roboterarms (2) vorgesehen ist, wobei die Drehvorrichtung (S) Folgendes umfasst:
eine Wellenabdeckung (7), die eine rohrförmige Wand (70) enthält, die sich in eine Axialrichtung (Da) erstreckt;
eine Drehwelle (9), die in das Innere der Wand (70) der Wellenabdeckung (7) eingesetzt wird und sich in Axialrichtung (Da) erstreckt;
ein Lager (8), das bewirkt, dass die Wellenabdeckung (7) die Drehwelle (9) trägt, sodass die Drehwelle (9) um eine Mittelachse der Drehwelle (9) drehbar ist; und
mindestens einen Arbeitsdraht (78), der innerhalb der Wand (70) der Wellenabdeckung (7) mit der Drehwelle (9) gekoppelt ist, wobei:
die Wellenabdeckung (7) eine Führung (74) enthält, die den mindestens einen Arbeitsdraht (78) führt;
wobei die Führung (74) dazu ausgebildet ist, um eine Symmetrieebene (P1), die durch eine Mittelachse der Wellenabdeckung (7) und parallel zur Axialrichtung (Da) verläuft, symmetrisch zu sein; und
wobei jeder Abschnitt der Führung (74), wobei sich die Abschnitte auf beiden Seiten der Symmetrieebene (P1) befinden, Folgendes enthält:
einen Außenrandabschnitt (743), der auf einer Außenfläche der Wand (70) ausgebildet ist und sich von einem Basisende der Wand (70) in Axialrichtung (Da) erstreckt,
einen Innenrandabschnitt (744), der entlang einer Innenfläche der Wand (70) ausgebildet ist und sich in Umfangsrichtung erstreckt, und
einen Verbindungsabschnitt (745), der den Außenrandabschnitt (743) und den Innenrandabschnitt (744) verbindet.

2. Drehvorrichtung (S) nach Anspruch 1, wobei der Verbindungsabschnitt (745) durch Folgendes ausgebildet wird:
eine Kommunikationsbohrung (746), die die Innenseite und Außenseite der Wand (70) durchdringt und sich auf dem Innenrandabschnitt (744) öffnet; und
eine Nut, die die Kommunikationsbohrung (746) und den Außenrandabschnitt (743) verbindet, in Form einer gekrümmten Linie, die auf der Außenfläche der Wand (70) vorgesehen ist.

3. Drehvorrichtung (S) nach Anspruch 2, wobei sich die Kommunikationsbohrung (746) von einem Öffnungsende der Kommunikationsbohrung (746) in Tangentialrichtung des Öffnungsendes erstreckt.

4. Drehvorrichtung (S) nach einem der Ansprüche 1 bis 3, wobei:
die Innenfläche der Wand (70) der Wellenabdeckung (7) eine ringförmige erste abgestufte Fläche (75), die in Axialrichtung (Da) einer Seite zugewandt ist, enthält;
eine Außenfläche der Drehwelle (9) eine ringförmige zweite abgestufte Fläche (93), die in Axialrichtung (Da) der anderen Seite zugewandt ist, enthält;
die erste abgestufte Fläche (75) und die zweite abgestufte Fläche (93) derart angeordnet sind, dass sie in Axialrichtung (Da) mit einem Abstand dazwischen einander zugewandt sind; und
der Innenrandabschnitt (744) zwischen der ersten abgestuften Fläche (75) und der zweiten abgestuften Fläche (93) durch eine Innenfläche der Wellenabdeckung (7) und die Außenfläche der Drehwelle (9) ausgebildet ist.

5. Drehvorrichtung (S) nach einem der Ansprüche 1 bis 4, mehrere Dreheinheiten (U1, U2) umfassend, die in Axialrichtung (Da) miteinander gekoppelt sind, wobei jede der Dreheinheiten (U1, U2) die Drehwelle (9), die Wellenabdeckung (7), das Lager (8) und den mindestens einen Arbeitsdraht (78) enthält.

6. Drehvorrichtung (S) nach Anspruch 5, ferner einen Verriegelungsmechanismus umfassend, der dazu ausgelegt ist, die Arbeitsdrähte (78) der mehreren Dreheinheiten (U1, U2) miteinander zu koppeln, um die mehreren Dreheinheiten (U1, U2) zusammen zu bedienen.

7. Medizinisches Instrument (1), Folgendes umfassend:
einen Roboterarm (2), der eine Drehvorrichtung (S) nach einem der Ansprüche 1 bis 6 umfasst; und
ein Greiforgan (24), das an einem Kopfende des Roboterarms (2) vorgesehen ist.

## Revendications

1. Dispositif de pivotement (S) prévu à une articulation d'un bras robotisé (2), le dispositif de pivotement (S) comprenant:
un couvercle d'axe (7) comprenant une paroi tubulaire (70) s'étendant dans une direction axiale (Da);
un axe de pivotement (9) inséré dans un côté interne de la paroi (70) du couvercle d'axe (7) et s'étendant dans la direction axiale (Da);
un palier (8) amenant le couvercle d'axe (7) à supporter l'axe de pivotement (9) de sorte que l'axe de pivotement (9) puisse tourner autour d'un axe central de l'axe de pivotement (9); et
au moins un câble de manipulation (78) couplé à l'axe de pivotement (9) au côté interne de la paroi (70) du couvercle d'axe (7), dans lequel:
le couvercle d'axe (7) comprend un guide (74) destiné à conduire l'au moins un câble de manipulation (78);
le guide (74) est formé de manière à être symétrique autour d'un plan de symétrie (P1) passant par un axe central du couvercle d'axe (7) et parallèle à la direction axiale (Da); et
chacune des parties du guide (74), lesquelles parties sont situées des deux côtés respectifs du plan de symétrie (P1), comprend
une partie périphérique externe (743) formée sur une surface externe de la paroi (70) et s'étendant depuis une extrémité de base de la paroi (70) dans la direction axiale (Da),
une partie périphérique interne (744) formée le long d'une surface interne de la paroi (70) et s'étendant dans une direction circonférentielle, et
une partie de connexion (745) reliant la partie périphérique externe (743) et la partie périphérique interne (744).

2. Dispositif de pivotement (S) selon la revendication 1, dans lequel la partie de connexion (745) est formée par:
un trou de communication (746) pénétrant dans un côté interne et un côté externe de la paroi (70) et débouchant sur la partie périphérique interne (744); et
une rainure reliant le trou de communication (746) et la partie périphérique externe (743) sous la forme d'une ligne courbe et prévue sur la surface externe de la paroi (70).

3. Dispositif de pivotement (S) selon la revendication 2, dans lequel le trou de communication (746) s'étend depuis une extrémité d'ouverture du trou de communication (746) dans une direction tangentielle de l'extrémité d'ouverture.

4. Dispositif de pivotement (S) selon l'une quelconque des revendications 1 à 3, dans lequel:
la surface interne de la paroi (70) du couvercle d'axe (7) comprend une première surface étagée annulaire (75) tournée vers un côté dans la direction axiale (Da);
une surface externe de l'axe de pivotement (9) comprend une seconde surface étagée annulaire (93) tournée vers l'autre côté dans la direction axiale (Da);
la première surface étagée (75) et la seconde surface étagée (93) sont agencées de manière à se faire face l'une l'autre avec un intervalle entre elles dans la direction axiale (Da); et
la partie périphérique interne (744) est formée entre la première surface étagée (75) et la seconde surface étagée (93) par une surface interne du couvercle d'axe (7) et la surface externe de l'axe de pivotement (9).

5. Dispositif de pivotement (S) selon l'une quelconque des revendications 1 à 4, comprenant une pluralité d'unités de pivotement (U1, U2) couplées les unes aux autres dans la direction axiale (Da), chacune des unités de pivotement (U1, U2) comprenant l'axe de pivotement (9), le couvercle d'axe (7), le palier (8), et l'au moins un câble de manipulation (78).

6. Dispositif de pivotement (S) selon la revendication 5, comprenant en outre un mécanisme de verrouillage configuré pour coupler les câbles de manipulation (78) de la pluralité d'unités de pivotement (U1, U2) les unes aux autres pour faire fonctionner la pluralité d'unités de pivotement (U1, U2) conjointement les unes aux autres.

7. Appareil médical (1) comprenant:
un bras robotisé (2) comprenant le dispositif de pivotement (S) selon l'une quelconque des revendications 1 à 6; et
un effecteur d'extrémité (24) prévu à une extrémité de pointe du bras robotisé (2).
